# EUROPEAN PATENT APPLICATION

(11) **EP 4 586 199 A1**
(43) Date of publication of application: **16.07.2025**
(21) Application number: 24222181.0
(22) Date of filing: 20.12.2024
(51) Int. Cl.: G06T 11/00, A61B 6/03

(54) **ADAPTIVE PIXEL ADJUSTMENT FOR SPECT IMAGING SYSTEM**

(30) Priority: 12.01.2024 US 202418412331
(71) Applicant: GE Precision Healthcare LLC, Waukesha, WI 53188 (US)
(72) Inventor: DIDI, Netanella, 3508415 Haifa (IL)
(74) Representative: Fennell, Gareth Charles

(57) **Abstract**

Methods and systems are provided for increasing a quality of images generated by a single photon emission computed tomography (SPECT) imaging system (100, 200). Photons penetrating a protective shielding (580) around detector heads (308, 408, 210) of the SPECT system (100, 200) may not be handled correctly by a reconstruction algorithm used to reconstruct an image from a plurality of projection views (701) acquired by the SPECT imaging system (100, 200), generating artifacts (1121, 1112, 730) and reducing a quality of the image. In one embodiment, the artifacts (1121, 1112, 730) may be reduced or eliminated by selectively applying the reconstruction algorithm to a first portion of pixel columns (1007, 1012) of the projection views (701) not including the artifacts (1121, 1112, 730), and not applying the reconstruction algorithm to a second portion of pixel columns (1007, 1012) of the projection views including the artifacts (1121, 1112, 730). A number of the second portion of pixel columns (1007, 1012) may be based on a sweep angle (550) of a detector (471) acquiring a projection view (701).

## Description

### TECHNICAL FIELD

Embodiments of the subject matter disclosed herein relate to improving image quality of single photon emission computed tomography (SPECT) imaging systems.

### BACKGROUND

Nuclear medicine (NM) imaging systems such as single photon emission computed tomography (SPECT) imaging systems may be used to diagnose certain disorders of a heart, brain, or other system of a patient. A radioactive substance may be introduced into the patient, which may be absorbed in a target organ or area of a body of the patient. The radioactive substance emits photons, which are collimated and detected by a detector subsystem. Detectors of the subsystem may generate output electrical signals from which three-dimensional (3D) images can be created, where the 3D images show a distribution of the radioactive substance in and around the target organ or area. Examining the distribution may aid a caregiver in diagnosing or monitoring a disorder of the patient. For example, the images may be used to diagnose clogged arteries, bone healing, seizures, cancer progression, or other problems.

A detector may be configured to detect photons entering collimators of the detector from within a specified angle range. The detector may include shielding around sides of the detector to prevent photons impinging on the detector from outside the specified angle range from being detected and counted. However, when high energy isotopes are used, some photons may penetrate the shielding and be detected. The penetrating photons may cause artifacts and/or reduce a quality of a reconstructed image.

### SUMMARY

The current disclosure at least partially addresses one or more of the above identified issues by a method for a single photon emission computed tomography (SPECT) imaging system, the method comprising acquiring a plurality of projection views of a scanned object using the SPECT imaging system, the plurality of projection views including artifacts; configuring a reconstruction algorithm to reconstruct an image based on a first portion of pixels of each projection view of the plurality of projection views, and not based on a second portion of pixels of each projection view of the plurality of projection views, the second portion of pixels including the artifacts; reconstructing an image using the reconstruction algorithm; and displaying the reconstructed image on a display device of the SPECT imaging system and/or storing the reconstructed image in a memory of the SPECT imaging system.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1A is a schematic block diagram of a nuclear imaging (NM) system, in accordance with an embodiment;
FIG. 1B is a schematic block diagram illustrating detector units, in accordance with an embodiment;
FIG. 1C is a pictorial view of an exemplary detector unit;
FIG. 2A shows a pictorial view of an exemplary multi-head SPECT imaging system, in accordance with one or more embodiments of the present disclosure;
FIG. 2B shows a pictorial view of an exemplary dual-head SPECT imaging system, in accordance with one or more embodiments of the present disclosure;
FIG. 3 illustrates an orientation of a plurality of detector units of a multi-head SPECT system during a scan of a phantom, in accordance with one or more embodiments of the present disclosure;
FIG. 4 shows an exemplary collimation of radiation from a phantom by a detector unit of a multi-head SPECT system, in accordance with one or more embodiments of the present disclosure;
FIG. 5 shows radiation penetrating shielding of the detector unit of FIG. 4, in accordance with one or more embodiments of the present disclosure;
FIG. 6 is a graph showing a relationship between shielding penetration and detector angle, in accordance with one or more embodiments of the present disclosure;
FIG. 7 shows an exemplary image reconstructed by a SPECT system including artifacts due to radiation penetration, in accordance with one or more embodiments of the present disclosure;
FIG. 8 is a flowchart illustrating an exemplary adaptive pixel zeroing method for removing artifacts from a CT image, in accordance with one or more embodiments of the present disclosure;
FIG. 9 is a graph illustrating the adaptive pixel zeroing as a function of sweep angle of a detector unit, in accordance with one or more embodiments of the present disclosure;
FIG. 10 is a block diagram illustrating how selected pixels of projection views may be ignored during image reconstruction using the adaptive pixel zeroing to reduce the artifacts of FIG. 7, in accordance with one or more embodiments of the present disclosure; and
FIG. 11 shows exemplary reconstructed images generated by the SPECT system using the adaptive pixel zeroing, in accordance with one or more embodiments of the present disclosure.

The drawings illustrate specific aspects of the described systems and methods. Together with the following description, the drawings demonstrate and explain the structures, methods, and principles described herein. In the drawings, the size of components may be exaggerated or otherwise modified for clarity. Well-known structures, materials, or operations are not shown or described in detail to avoid obscuring aspects of the described components, systems and methods.

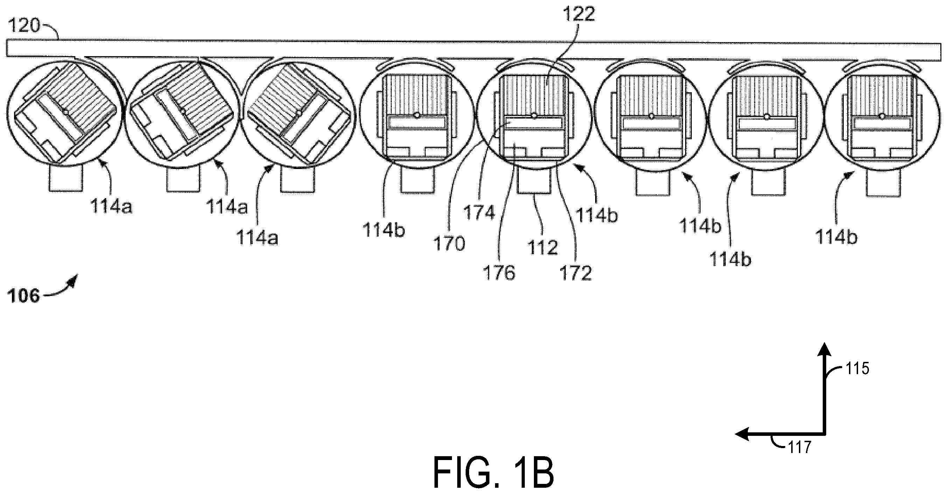

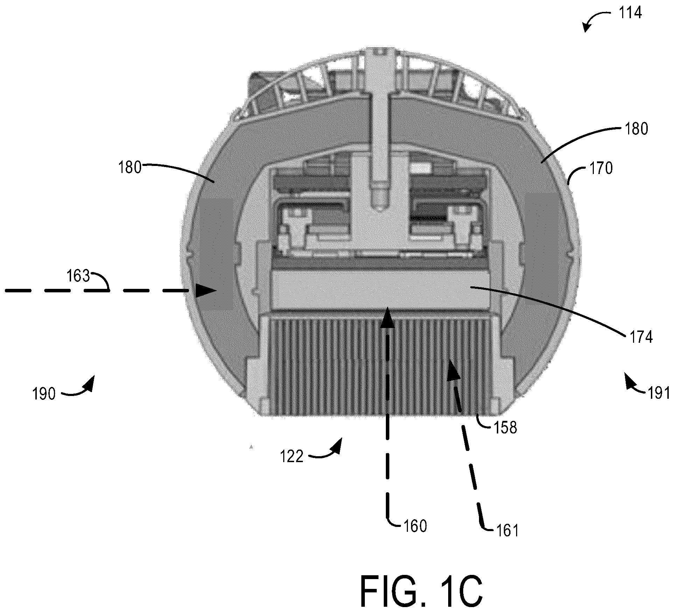

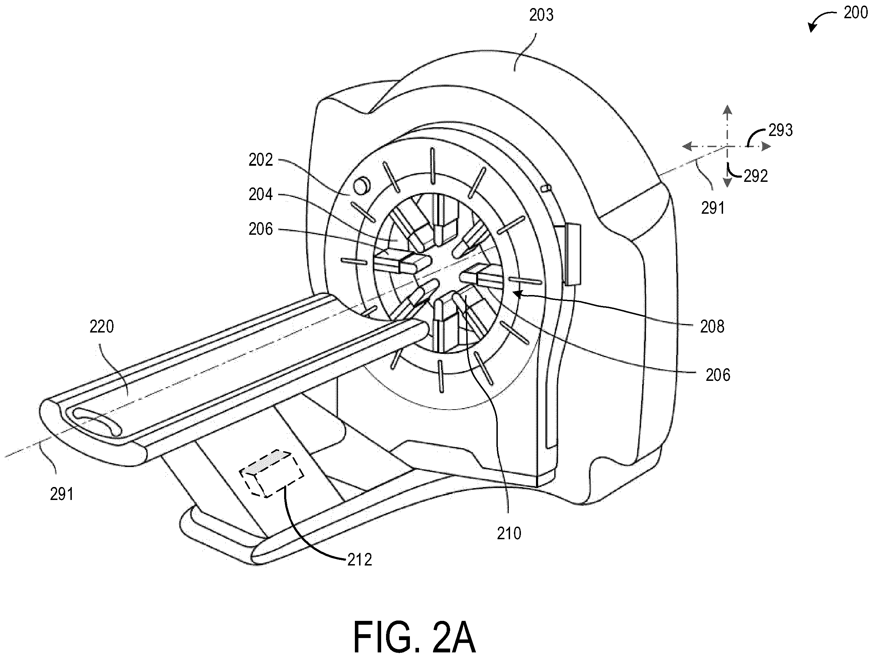

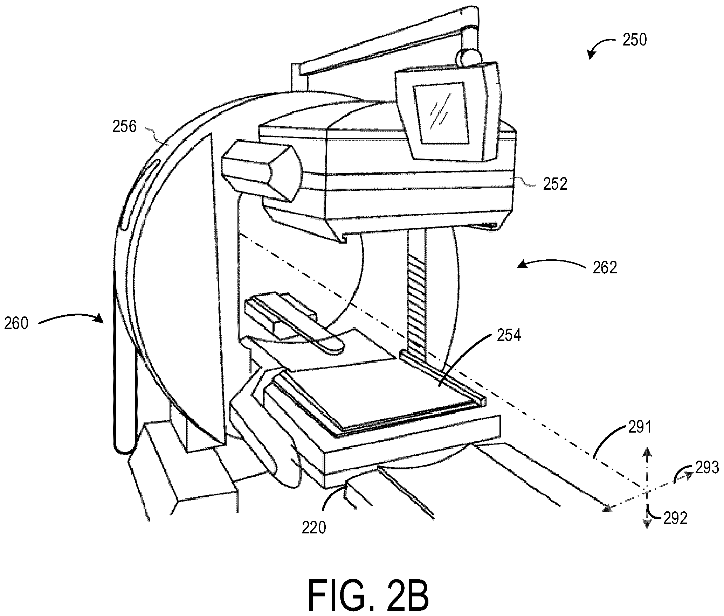

FIGS. 1B, 1C, 2A, and 2B show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space therebetween and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

### DETAILED DESCRIPTION

This description and embodiments of the subject matter disclosed herein relate to methods and systems for a single photon emission computed tomography (SPECT) system. In particular, methods and systems are proposed to increase an image quality of images reconstructed using the SPECT system.

A SPECT system shows a distribution of a radioactive substance inside a patient's body. A radioactive tracer (e.g., a source of penetrating radiation) is administered to the patient, which typically includes a pharmaceutical tagged with a radionuclide which emits radiation photons (radiopharmaceutical). The radiopharmaceutical is designed to be absorbed in a target organ, such as the heart muscle, or other organs or body part of interest. One or more detector arrays may be rotated about a gantry within an imaging plane and around the patient, and images are generated from the radiation photons at a plurality of views at different view angles. Additionally, some SPECT systems may include detector arrays with multiple detector heads, where each detector head may rotate or pivot, in addition to the gantry rotation. The emitted radiation photons are collimated with a collimator subsystem and detected by a detector subsystem which generates output electrical signals. The electrical signals are digitized and processed by a computer system to generate images of a regional distribution of the radioactive sources in and around the target organ.

As a detector head rotates and/or pivots, photons emitted by the radiopharmaceutical within a range of view angles (also referred to herein as detector sweep angles or sweep angles) of the detector head are focused on detector elements by a collimator. Photons directed at the detector head outside the view angle range may impinge on a side of the detector head and may not be collimated and detected. The side of the detector head may include shielding to prevent the non-collimated photons from being detected. However, when high energy isotopes are used, some photons may penetrate the shielding and be detected. The penetrating photons may cause artifacts and/or reduce a quality of a reconstructed image. For example, a reconstruction algorithm used by the SPECT system may misinterpret the penetrating photons, and generate artifacts along a contour of a scanned object. An additional effect of the penetrating photons may be that a quality and/or visibility of internal structures or features of the scanned object may be reduced.

The penetrating photons may be prevented by increasing a thickness of the shielding. However, the shielding may include a heavy and/or dense material, such as lead, and increases in the thickness of the shielding increases a weight and size of the detector. Increases in the size of the detector may restrict a motion of the detector and affect how close the detector can be positioned next to the scanned object. Increases in the weight of the detector may increase a cost of engines used to control the detector and may restrict a movement of the detector. As a result, it is desirable to minimize the thickness of the shielding, and mitigate the penetrating photons in a different manner.

To address this issue, systems and methods are proposed to reduce the effect of penetrating radiation algorithmically during image reconstruction. Specifically, one or more sets of adjacent pixels, such as pixel columns on a side of each projection, may be ignored using a mask when applying a reconstruction algorithm. In addition, for multi-head detectors, since shielding penetration is higher in views acquired at large angles and lower in views acquired at small angles, a number of adjacent pixels and/or pixel columns that are ignored may be selected in an adaptive manner, based on the angle (e.g., the larger the angle, the more pixels columns ignored).

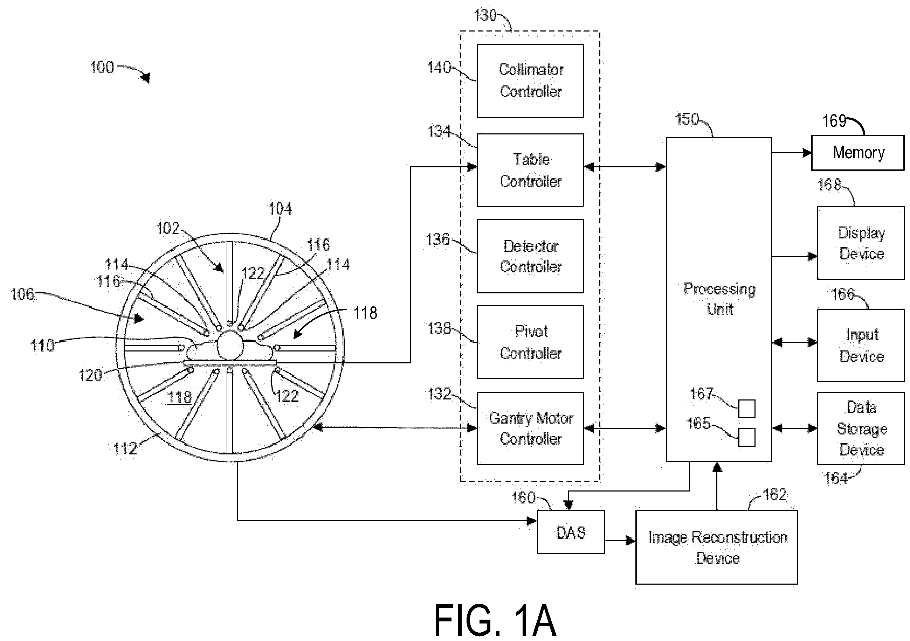

FIG. 1A is a schematic illustration of a NM imaging system 100 having a plurality of imaging detectors mounted on a gantry. The imaging detectors may be configured to rotate around a fixed pivot. The movement of the imaging detectors is controlled to reduce the likelihood or avoid collision among the moving imaging detectors and/or reduce the likelihood of one imaging detector obstructing the field of view of another imaging detector. For example, the NM imaging system in some embodiments provides coordinated swinging or rotating motion of a plurality of imaging detectors or detector heads.

In particular, a plurality of imaging detectors 102 are mounted to a gantry 104 and/or a patient support structure (not shown) (e.g., under a patient table 120), which may define a table support for a patient table 120. In the illustrated embodiment, the imaging detectors 102 are configured as a detector array 106 positioned around the subject 110 (e.g., a patient), as viewed in FIG. 1A. The detector array 106 may be coupled directly to the gantry 104, or may be coupled via support members 112 thereto, to allow movement of the entire array 106 relative to the gantry 104 (e.g., rotational movement in the clockwise or counterclockwise direction as viewed in FIG. 1A). Additionally, each of the imaging detectors 102 includes a detector unit 114, at least some of which are mounted to a movable detector carrier 116 (e.g., a support arm or actuator that may be driven by a motor to cause movement thereof) that extends from the gantry 104. For the purposes of this disclosure, the detector units may also be referred to as detector heads. In some embodiments, the detector carriers 116 allow movement of the detector units 114 towards and away from the subject 110, such as linearly. Thus, in the illustrated embodiment the detector array 106 is around the subject 110 and may allow linear movement of the detector units 114, such as towards or away from the patient table 120 in one embodiment. However, other configurations and orientations are possible as described herein, as well as different types of movements (e.g., transverse or perpendicular movement relative to the patient table 120). It should be noted that the movable detector carrier 116 may be any type of support that allows movement of the detector units 114 relative to the support member 112 and/or gantry 104, which in various embodiments allows the detector units 114 to move linearly towards and away from the support member 112, such as radially inward and outwards for positioning adj acent the subject 110. For example, as described herein, the detector units 114 may be controlled to move independently of each other towards or away from the subject 110, as well as capable of rotational, pivoting, or tilting movement in some embodiments.

Each of the imaging detectors 102 in various embodiments is smaller than a conventional whole body or general purpose imaging detector. A conventional imaging detector may be large enough to image most or all of a width of a patient's body at one time and may have a diameter of approximately 50 cm or more. In contrast, each of the imaging detectors 102 may include one or more detector units 114 coupled to a respective detector carrier 116 and having dimensions of 4 cm to 20 cm and may be formed of Cadmium Zinc Telluride (CZT) tiles or modules. For example, each of the detector units 114 may be 8x8 cm in size and be composed of a plurality of CZT pixelated modules (not shown). For example, each module may be 4x4 cm in size and have 16x16=256 pixels. In some embodiments, each detector unit 114 includes a plurality of modules, such as an array of 1x7 modules. However, different configurations and array sizes are contemplated including, for example, detector units 114 having multiple rows of modules.

It should be understood that the imaging detectors may be different sizes and/or shapes with respect to each other, such as square, rectangular, circular, or another shape. An actual field of view (FOV) of each of the imaging detectors 102 may be directly proportional to the size and shape of the respective imaging detector.

The gantry 104 may be formed with an aperture 118 (e.g., opening or bore) therethrough as illustrated. The patient table 120 is configured with a support mechanism, such as the patient support structure, to support and carry the subject 110 in one or more of a plurality of viewing positions within the aperture 118 and relative to the imaging detectors 102. Alternatively, the gantry 104 may comprise a plurality of gantry segments (not shown), each of which may independently move a support member 112 or one or more of the imaging detectors 102.

The gantry 104 may also be configured in other shapes, such as a "C", "H", and "L", for example, and may be rotatable about the subject 110. For example, the gantry 104 may be formed as a closed ring or circle, or as an open arc or arch which allows the subject 110 to be easily accessed while imaging and facilitates loading and unloading of the subject 110, as well as reducing claustrophobia in some subjects 110. For example, in some embodiments the gantry 104 may be arc shaped and the support members 112 movable along the arc to position the detector units 114 at different locations along the gantry 104. In some embodiments, the detector units 114 may also be independently movable along the gantry 104. For example, in some embodiments, NM imaging system 100 may be configured in an alternate dual-head configuration including two detector arrays, each including a set of detector units 114. The two detector arrays may be positioned on opposite sides of an open-arch gantry 104, as described below in reference to FIG. 2B.

Additional imaging detectors (not shown) may be positioned to form rows of detector arrays or an arc or ring around the subject 110. By positioning multiple imaging detectors 102 at multiple positions with respect to the subject 110, such as along an imaging axis (e.g., head to toe direction of the subject 110), image data specific for a larger FOV may be acquired more quickly.

Each of the imaging detectors 102 has a radiation detection face, which is directed towards the subject 110 or a region of interest within the subject 110. The radiation detection faces may be covered by or have coupled thereto a collimator 122. The actual FOV for each of the imaging detectors 102 may be increased, decreased, or relatively unchanged by the type of collimator 122. In one embodiment, the collimator 122 is a multi-bore collimator, such as a parallel-hole collimator. However, other types of collimators, such as converging or diverging collimators may optionally or alternatively be used. Other examples for the collimator 122 include pinhole, parallel-beam converging, diverging fan-beam, converging or diverging cone-beam, multi-bore converging, multi-bore converging fan-beam, multi-bore converging cone-beam, multi-bore diverging, or other types of collimators.

Optionally, multi-bore collimators may be constructed to be registered with pixels of the detector units 114, which in one embodiment are CZT detectors. However, other materials may be used. Registered collimation may increase spatial resolution by forcing photons going through one bore to be collected primarily by one pixel. Additionally, registered collimation may increase sensitivity and energy response of pixelated detectors as detector area near the edges of a pixel or in between two adjacent pixels may have reduced sensitivity or decreased energy resolution or other performance degradation. Having collimator septa directly above the edges of pixels reduces the chance of a photon impinging at these degraded performance locations, without decreasing the overall probability of a photon passing through the collimator.

A controller unit 130 may control the movement and positioning of the patient table 120, imaging detectors 102, gantry 104, and/or the collimators 122. A range of motion before or during an acquisition, or between different image acquisitions, is set to maintain the actual FOV of each of the imaging detectors 102 directed, for example, towards or "aimed at" a particular area or region of the subject 110 or along the entire subject 110.

The controller unit 130 may have a gantry motor controller 132, table controller 134, detector controller 136, pivot controller 138, and collimator controller 140. The controllers 130, 132, 134, 136, 138, 140 may be automatically commanded by a processing unit 150, manually controlled by an operator, or a combination thereof. The gantry motor controller 132 may move the imaging detectors 102 with respect to the subject 110, for example, individually, in segments or subsets, or simultaneously in a fixed relationship to one another. For example, in some embodiments, the gantry controller 132 may cause the imaging detectors 102 and/or one or more of the support members 112 to rotate about the subject 110, which may include motion of less than or up to 180 degrees (or more).

The table controller 134 may move the patient table 120 to position the subject 110 relative to the imaging detectors 102. The patient table 120 may be moved in up-down directions, in-out directions, and right-left directions, for example. The detector controller 136 may control movement of each of the imaging detectors 102 to move closer to and farther from a surface of the subject 110, such as by controlling translating movement of the detector carriers 116 linearly towards or away from the subject 110 (e.g., sliding or telescoping movement). Optionally, the detector controller 136 may control movement of the detector carriers 116 to allow coordinated movement of the detector array 106.

The pivot controller 138 may control pivoting, rotating, or swinging movement of the detector units 114 at ends of the detector carriers 116, and/or the detector carrier 116. For example, one or more of the detector units 114 or detector carriers 116 may be rotated or swung about at least one axis to view the subject 110 from a plurality of angular orientations. The collimator controller 140 may adjust a position of an adjustable collimator, such as a collimator with adjustable strips (or vanes) or adjustable pinhole(s).

It should be noted that motion of one or more imaging detectors 102 may be in directions other than strictly axially or radially, and optionally, motions in several motion directions may be used. Moreover, the motions of the imaging detectors 102 are coordinated in various embodiments as described herein. Therefore, the term "motion controller" may be used to indicate a collective name for all motion controllers. It should be noted that the various controllers may be combined, for example, the detector controller 136 and pivot controller 138 may be combined to provide the different movements described herein.

Prior to acquiring an image of the subject 110 or a portion of the subject 110, the imaging detectors 102, gantry 104, patient table 120, and/or collimators 122 may be adjusted as discussed in more detail herein, such as to first or initial imaging positions, as well as subsequent imaging positions. The imaging detectors 102 may each be positioned to image a portion of the subject 110. Alternatively, one or more of the imaging detectors 102 may not be used to acquire data, such as the imaging detectors 102 at ends of the detector array 106, which as illustrated in FIG. 1A are in a protracted position towards the subject 110. Positioning may be accomplished manually by the operator and/or automatically, which may include using other images acquired before the current acquisition, such as by another imaging modality such as CT, MRI, X-ray, PET, or ultrasound. Additionally, the detector units 114 may be configured to acquire non-NM data, such as x-ray CT data.

After the imaging detectors 102, gantry 104, patient table 120, and/or collimators 122 are positioned, one or more images are acquired by one or more of the imaging detectors 102 being used, which may include pivoting or swinging motion of one or more of the detector units 114, which may pivot, rotate, or swing to different degrees or between different ranges of angles. The image data acquired by each imaging detector 102 may be combined and reconstructed into a composite image, which may comprise two-dimensional (2D) images, a three-dimensional (3D) volume, or a 3D volume over time (4D).

In one embodiment, the imaging detectors 102, gantry 104, patient table 120, and/or collimators 122 remain stationary after being initially positioned. In another embodiment, an effective field of view for one or more of the imaging detectors may be increased by movement such as pivoting, rotating, or swinging one or more of the imaging detectors 102, rotating the detector array 106 with the gantry 104, adjusting one or more of the collimators 122, or moving the patient table 120.

In various embodiments, a data acquisition system (DAS) 160 receives electrical signal data produced by the imaging detectors 102 and converts this data into digital signals for subsequent processing. An image reconstruction device 162 and a data storage device 164 may be provided in addition to the processing unit 150. It should be noted that one or more functions related to one or more of data acquisition, motion control, data processing, and image reconstruction may be accomplished through hardware, software, and/or by shared processing resources, which may be located within or near the imaging system 100, or may be located remotely. Additionally, a user input device 166 may be provided to receive user inputs (e.g., control commands), as well as a display 168 for displaying images.

Additionally, a detector position controller 165 is also provided, which may be implemented in hardware, software, or a combination thereof. For example, as shown in FIG. 1A, the detector position controller 165 may form part of or operate in connection with the processing unit 150. In some embodiments, the detector position controller 165 may be a module that operates to control the movement of the imaging detectors 102, including the detector units 114, such that coordinated or synchronized movement is provided as described herein. It should be noted that movement of a plurality of the imaging detectors 102 and/or detector units 114 may be performed at the same time (e.g., simultaneously or concurrently) or at different times (e.g., sequentially or step-wise, such as back and forth between two detector units 114). It also should be understood that when referring to a detector head, such a detector head may include one or multiple detector modules.

At least the processing unit 150 may be electronically coupled to a non-transitory memory 169. The memory 169 may comprise any known data storage medium. In some embodiments, the memory 169 may include components disposed at two or more devices, which may be remotely located and/or configured for coordinated processing. In some embodiments, one or more aspects of the memory 169 may include remotely-accessible networked storage devices configured in a cloud computing configuration. The memory 169 may store information for configuring NM imaging system 100. In one example, the memory 169 may store reference or lookup tables used to select, adjust, and/or configure a reconstruction algorithm of NM imaging system 100, or a different algorithm.

In operation, and as shown, for example, in FIG. 1B, one embodiment includes the detector array 106 positioned (e.g., mounted) under the patient table 120. As can be seen, a plurality of detector units 114a, 114b are positioned in adjacent arrangement, for example, along one or more rows under the patient table 120 (it should be noted that only a single row of detector units is shown). The detector units in some embodiments are aligned along one or more axes generally perpendicular to the longitudinal axis of the patient table 120, which defines an examination axis (e.g., from head to toe of the subject 110). However, it should be appreciated that the detector units may be aligned in different configurations and orientations, which may be offset from each other, transverse to the longitudinal axis of the patient table 120 and/or parallel to the longitudinal axis of the patient table 120. The detector units illustrated in FIG. 1B may each be non-limiting examples of detector unit 114 of FIG. 1A. Further, detector units 114a illustrated in FIG. 1B are arranged at an angle relative to a longitudinal axis 115 of the detector units (which is perpendicular to a longitudinal axis 117 of the patient table) and detector units 114b illustrated in FIG. 1B are arranged parallel to the longitudinal axis 115 of the detector units. In other embodiments, the detector units 114 may be positioned/mounted in detector heads arranged on sides of the patient table 120, such as in the dual-head SPECT system depicted in FIG. 2B.

As can be seen in FIG. 1B, each of the detector units 114a, 114b includes a housing 170, which are illustrated as a circular housing. However, the housing 170 of the detector units 114a, 114b may have different shapes and sizes, for example, oval, other curved shapes, etc. The detector units 114a, 114b include within the housing 170 a detector support 172, which may be a frame or other support structure. A detector 174 is coupled to the detector support 172. For example, the detector 174 may include one or more CZT tiles or modules as described herein, which are connected to electronics 176 (e.g., output electronics to output detected events) therein. Additionally, the collimator 122 is mounted to a front detecting surface of the detector 174. Thus, the detector support 172 is sized and shaped, such as having a base and/or walls, to support and maintain the components of the detector unit 114a, 114b within the housing 170. For example, the components of the detector unit 114a, 114b are maintained within the housing 170 when the housing rotates, pivots, or swings as described in more detail herein. In the illustrated embodiment, the detector units 114a are shown in a rotated, pivoted, or swung position, while the detector units 114b are shown in a non-rotated, non-pivoted, or non-swung position. As can be seen, in the non-rotated, non-pivoted, or non-swung position, the detecting face of the detector is generally parallel to the patient support surface of the patient table 120, while in the rotated, pivoted, or swung position, the detecting face of the detector is not parallel to the patient support surface of the patient table 120. Various embodiments provide coordinated or synchronized movement of the detector units 114a, 114b, which allows the detector units 114a, 114b to be positioned or packed in closer alignment than in conventional systems. For example, in some embodiments, different detector units 114a, 114b, such as adjacent detector units 114 may move along different angular ranges, to a different angular position, and/or at different velocities.

It should be noted that the arrangement of detector units 114 in the detector array 106 may be provided in other portions of the NM imaging system 100, such as at positions along the gantry 104 or as part of the detector array 106. Also, it should be noted that in some embodiments, a housing 170 is not provided surrounding or encasing the components within the detector units 114.

As seen in FIG. 1B, the housings 170 for detector units 114 are generally circular in shape and lie in close proximity to each other. As such, rotation of each detector unit 114 about its individual axis does not physically interfere with the adjacent detector units. The circular housings 170 allow for a small clearance in between each detector unit 114 to allow for complete rotation of each detector unit during operation of NM imaging system 100.

FIG. 1C shows a more detailed view of a detector unit 114, including the detector 174 positioned behind the collimator 122. The collimator 122 includes a plurality of parallel septa 158, which may allow photons directed towards the detector 174 at a perpendicular or close to perpendicular angle to impinge on the detector 174, as shown by dashed arrow 160. However, the septa 158 may prevent photons directed towards the detector 174 at a non-perpendicular angle (shown by dashed arrow 161) from impinging on the detector 174.

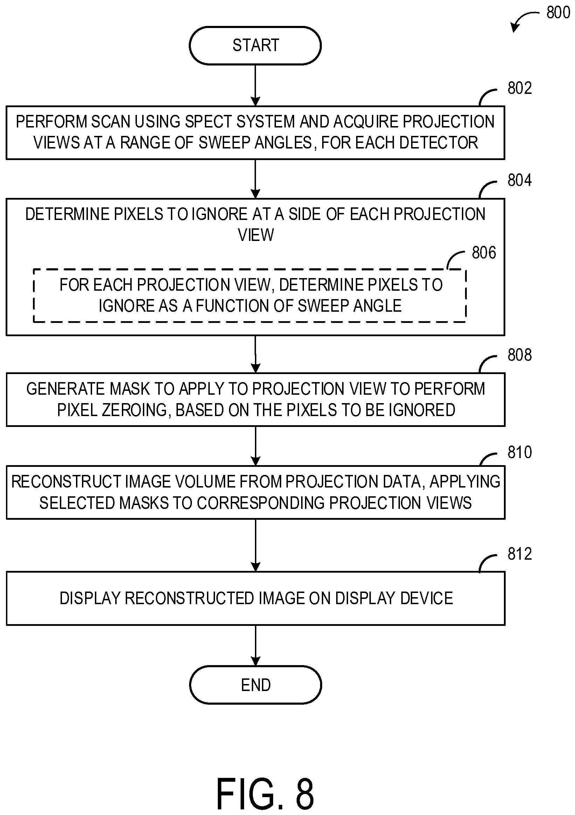

Additionally, the detector unit 114 may include a protective shielding 180, which may surround and/or enclose the detector 174. In FIG. 1C, the protective shielding 180 is depicted on a first side 190 and a second side 191 of the detector unit 114. However, it should be appreciated that the protective shielding 180 may encircle the detector 174 in three dimensions, such that photons directed towards the detector 174 from sides of detector unit 114 are prevented from being detected by the detector 174, as shown by the dashed arrow 163. The protective shielding 180 may be made of a heavy or dense material, such as, for example, lead. Nevertheless, when high energy isotopes are used, some photons may penetrate through the protective shielding 180 and be detected and counted by the detector 174. Because these penetrating photons are not collimated, they may not be handled appropriately by a reconstruction algorithm used to reconstruct an image from projection data acquired by the detector unit 114. As a result, the penetrating photons may decrease a quality of the reconstructed image, as described in greater detail below. Additionally, the penetrating photons may cause artifacts in the reconstructed image. The artifacts may be reduced and the image quality may be increased by following the method of FIG. 8 described below.

FIG. 2A is a perspective view of a first embodiment of a nuclear medicine (NM) imaging system 200. The NM imaging system 200 includes elements that are similar or identical to the elements of the NM imaging system 100. It should be noted that the arrangement of FIG. 2A is provided by way of example for illustrative purposes, and that other arrangements may be employed in various embodiments. The NM imaging system 200 of FIG. 2A is configured as a SPECT imaging system. In the illustrated embodiment, the NM imaging system 200 has a gantry 202 including a cavity 204 that is sized and shaped to receive an object therein. In particular embodiments, the object is a patient (e.g., human or animal). The cavity 204 is oriented relative to mutually perpendicular longitudinal, vertical, and horizontal axes 291, 292, 293. The cavity 204 extends lengthwise along the longitudinal axis 291. In the illustrated embodiment, the longitudinal axis 291 is a central longitudinal axis that extends through a geometric center of the cavity 204. The vertical axis 292 extends parallel to a gravitational force in FIG. 2A. However, for other configurations of the NM imaging system, the vertical axis 292 may not extend parallel to the gravitational force. Optionally, the NM imaging system 100 may adjoin or be positioned adjacent to a computed tomography (CT) imaging system (not shown). The gantry 202 has a discrete housing 203 and is configured to rotate at a rotational speed in one or both directions about the longitudinal axis 291.

The NM imaging system 200 also includes a plurality of detector assemblies 206 (e.g., detector units 114 of FIGS. 1A and 1B). As shown, the detector assemblies 206 are positioned in an array 208 in which the detector assemblies 206 are distributed at least partially around the cavity 204. In the illustrated embodiment, the detector assemblies 206 are evenly distributed circumferentially about the longitudinal axis 291. Each of the detector assemblies 206 in the array 208 includes a movable arm (not shown) and a detector head 210 that is coupled to the movable arm. The movable arm is configured to move the detector head 210 toward and away from the object within the cavity 204.

Also shown, the NM imaging system 200 includes a movable table 220 (e.g., patient table 120). The movable table 220 is configured to receive the object (e.g., a patient) and move the object into the cavity 204 along the longitudinal axis 291. In some embodiments, the movable table 220 may move in one or both directions along the vertical axis 292 and in one or both directions along the horizontal axis 293. Movement along the vertical axis 292 and the horizontal axis 293 may occur simultaneously or in separate movements (e.g., first up, then over). As set forth herein, the NM imaging system 200 may move the detector heads 210 and the movable table 220 so that a series of detector heads 210 are positioned in a dense group that borders the object.

The movable table 220 is operably coupled to one or more motors 212 that are controlled by one or more processors (not shown). The motors 212 are configured to move the movable table 220 to a designated position. For example, the processor(s) may control the motors 212 and the detector assemblies 206 so that the object has a desired position relative to the detector heads 210.

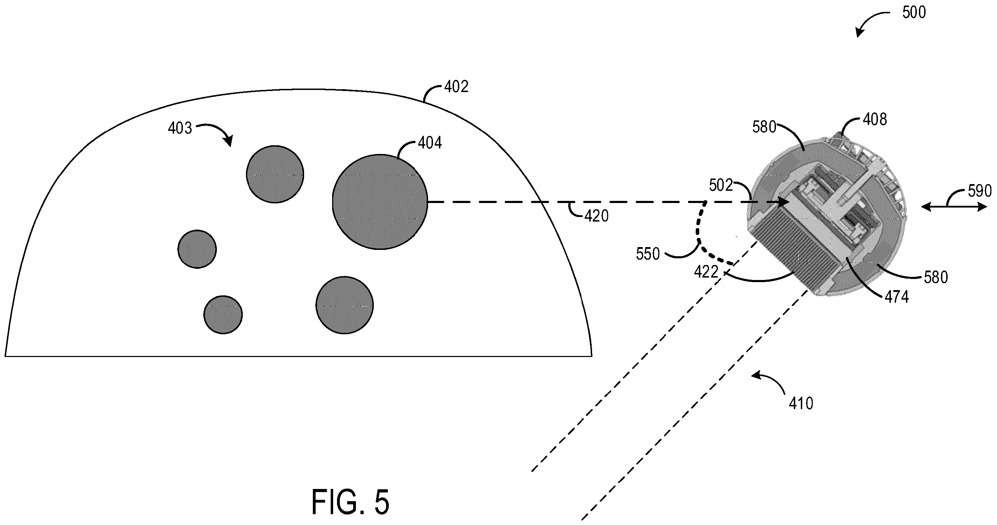

FIG. 2B shows a second embodiment of NM imaging system 200, with an alternate, dual-head configuration 250. In dual-head configuration 250, a first head 252 includes a first detector array with a first set of detector units (e.g., detector units 114 of FIG. 1A), and a second head 254 includes a second detector array with a second set of detector units. First head 252 and second head 254 may be arranged on a gantry 256 (e.g., gantry 104), which may be an open-arch gantry. As described above in reference to FIG. 1A, gantry 256 may rotate around a subject positioned on a movable table 220 (e.g., patient table 120). In FIG. 2B, first head 252 is aligned horizontally above movable table 220, and second head 254 is aligned horizontally below movable table 220, with respect to vertical axis 292. Alternatively, gantry 256 may be rotated such that first head 252 is aligned vertically at a first side 260 of movable table 220, and second head 254 is aligned vertically at a second, opposite side 262 of movable table 220, with respect to horizontal axis 293. In various embodiments, first head 252 and second head 254 may be aligned vertically during a calibration of NM imaging system 100 using a line source, as described in greater detail below in reference to FIG. 5. An angle between first head 252 and second head 254 may be preferably adjustable between 90 degrees and 180 degrees, using conventional means, and/or a distance between first head 252 and second head 254 may be adjusted and a transverse position of each of first head 252 and second head 254 (or of both together) may be adjusted, using conventional mechanical structures. Additionally, movable table 220 may be adjusted up or down along vertical axis 292, for example, to center a subject between first head 252 and second head 254. First head 252 and second head 254 may also be adjusted up or down along vertical axis 292, for example, to reduce or increase a distance between either or both of first head 252 and second head 254 and the subject.

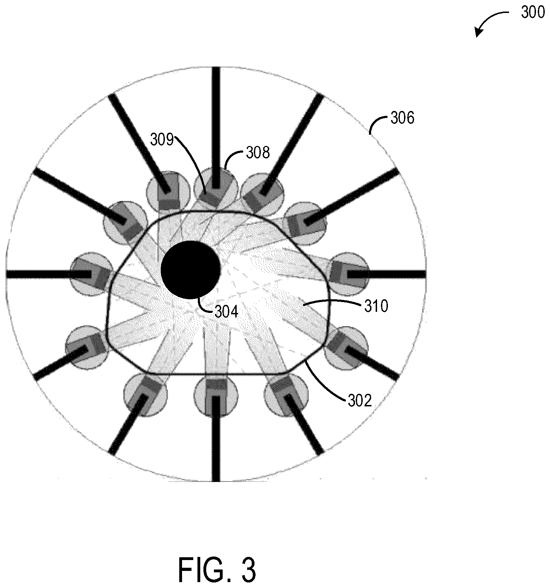

FIG. 3 shows a front view perspective of an exemplary configuration of a plurality of detector heads 308 (e.g., detector units 114) of a SPECT system 300, which may be a non-limiting example of NM imaging system 100 of FIG. 1A. In the depicted embodiment, the SPECT system 300 is a multi-head configuration, such as the multi-head configuration of FIG. 2A, where the plurality of detector heads 308 are moveably coupled to a gantry 306 (e.g., gantry 104). In other embodiments, the SPECT system may be a dual-head configuration, such as the dual-head configuration 250 of FIG. 2B, where the plurality of detector heads may be arranged linearly as depicted in FIG. 1B. In the depicted embodiment, the SPECT system is depicted during a scan of a phantom 302 that includes a high contrast region 304. The high contrast region 304 may be injected with a radioactive tracer, which may emit radiation (e.g., photons) that may be detected by detector elements.

Each detector head 308 may include a detector array (e.g., detector 174) with a plurality of detector elements. Each detector element may generate an electrical signal when a photon is detected by the element, and the electrical signals from the plurality of detector elements may be combined to generate a 3D image of the phantom 302. The detector heads 308 may each be collimated via a collimator 309, such that radiation emitted by the high contrast region 304 and received at a perpendicular angle to collimator 309 may be detected at the detector elements of a respective detector head 308. Each detector head 308 may rotate, such that each detector head 308 may sweep across the phantom 302 at a plurality of sweep angles. Each detector head 308 has a field of view 310 at each sweep angle, indicated in FIG. 3 as a shaded area between two lines extending out from the collimator 309 of each detector head 308.

As each detector head 308 sweeps across the phantom 302, the high contrast region 304 may be within the field of view 310 of some detector heads 308, and may be out of the field of view 310 of other detector heads 308. When the high contrast region 304 is within the field of view 310 of a detector head 308, photons emitted by the high contrast region 304 may be collimated by the collimator 309 and may be detected by the detector array of the detector head 308. When the high contrast region 304 is outside the field of view 310 of a detector head 308, photons directed at the detector head 308 from the high contrast region 304 may be absorbed by shielding of the detector head 308 (e.g., the protective shielding 180 of FIG. 1C), and may not be detected at the detector head 308.

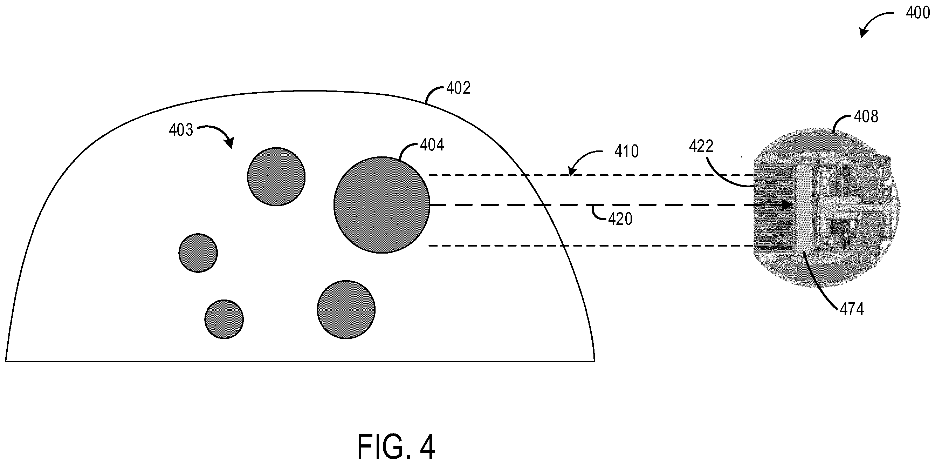

The role of the shielding is clarified by FIGS. 4 and 5. Turning to FIG. 4, a first diagram 400 shows a detector head 408, which may be a non-limiting example of the detector head 308 of FIG. 3. The detector head 408 is shown in a first position with respect to a phantom 402 (e.g., the phantom 302), which includes various high contrast regions 403 where a radioactive tracer has been injected. As a result of the injected radioactive tracer, the high contrast regions 403 may emit photons. In the first position, a high contrast region 404 (e.g., the high contrast region 304) is within a field of view 410 (e.g., field of view 310) of the detector head 408. The field of view 410 may widen as a function of distance from the detector head. As a result of being within the field of view 410, a photon emitted by the high contrast region 404 following a trajectory indicated by a dashed arrow 420 may be collimated by a collimator 422 of detector head 408 (e.g., collimator 122 of FIG. 1C), and a detector 474 of the detector head 408 may detect the photon.

In contrast, FIG. 5 shows a second diagram 500, where the detector head 408 is in a second position with respect to the phantom 402. In the second position, the detector head 408 is at a sweep angle 550 with respect to a longitudinal axis 590 of the detector head 408 (e.g., the longitudinal axis 115 of FIG. 1B). As such, the high contrast region 404 is not within the field of view 410. Because the detector head 408 is at the sweep angle 550 with respect to the high contrast region 404, a photon following the trajectory indicated by the dashed arrow 420 may impinge on the detector head 408 at a location 502 on an exterior surface of the detector head 408. As a result, the photon may be attenuated by a shielding 580 of the detector head 408 (e.g., protective shielding 180 of FIG 1C), and may not be detected at the detector 474.

However, in some cases, a portion of photons following trajectories similar to dashed arrow 420 may penetrate the shielding 580, and may not be attenuated by the shielding 580. When the photons penetrate the shielding 580, the photons may be detected and counted at the detector 474. Further, a number of photons that penetrate the shielding 580 may depend on the sweep angle 550. That is, as the sweep angle 550 increases, the number of photons penetrating the shielding 580 may increase, and as the sweep angle 550 decreases, the number of photons penetrating the shielding 580 may decrease.

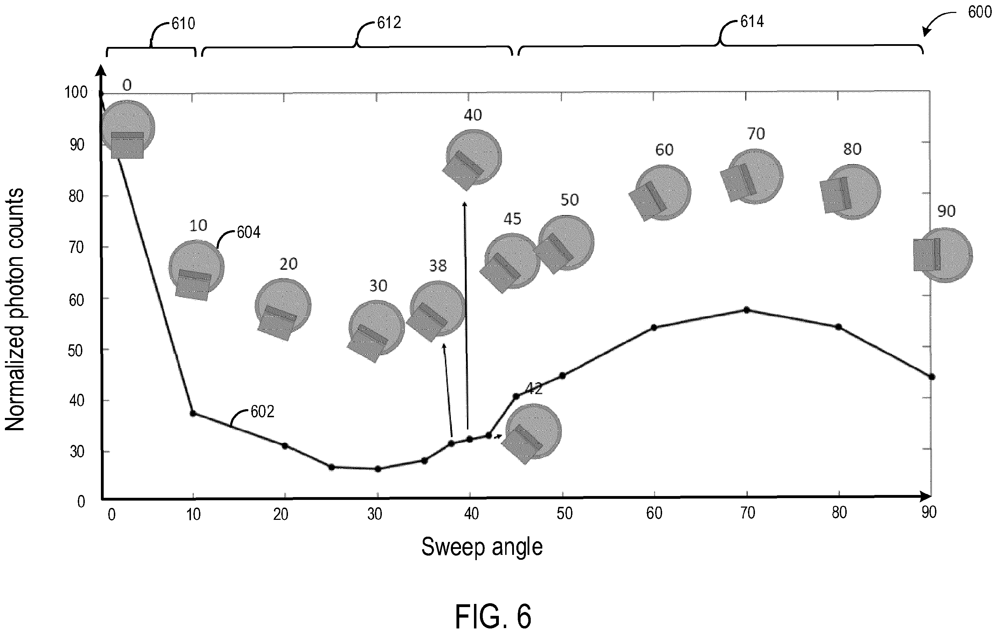

Referring to FIG. 6, a sweep angle graph 600 includes a plot 602 illustrating how a number of normalized photons counted at a detector head may vary with a sweep angle of the detector head. Normalized photon counts are depicted on a vertical axis of graph 600, and an angle of the detector head with respect to incoming photons emitted by a radioactive tracer. (e.g., the sweep angle 550) is indicated on a horizontal axis of sweep angle graph 600. A pictorial representation 604 of the detector head (e.g., detector heads 308 and 408) shows an orientation of the detector head at various points of interest along plot 602.

At a sweep angle of 0°, the detector head is aligned with a trajectory of the photon (e.g., trajectory 420), and the normalized number of photon counts is highest, meaning close to 100. During a first portion 610 of plot 602, as the sweep angle increases from 0° to 10°, the normalized number of photon counts decreases as the incoming photons move out of a field of view of the detector head. During a second portion 612 of plot 602, as the sweep angle increases from 10° to about 40°, the normalized number of photon counts continues to be low, as less photons are collimated and detected at the detector head. However, during a third portion 614 of plot 602, as the sweep angle of the detector head increases from 45° to about 90°, the normalized number of photon counts increases. The increase in photon counts as the sweep angle approaches 90° is due to photons striking a side of the detector head and penetrating shielding of the detector head. The photons penetrating the shielding of the detector head may generate artifacts in projection views acquired by the detector head, and generally decrease a quality of an image reconstructed from the projection views, as described above.

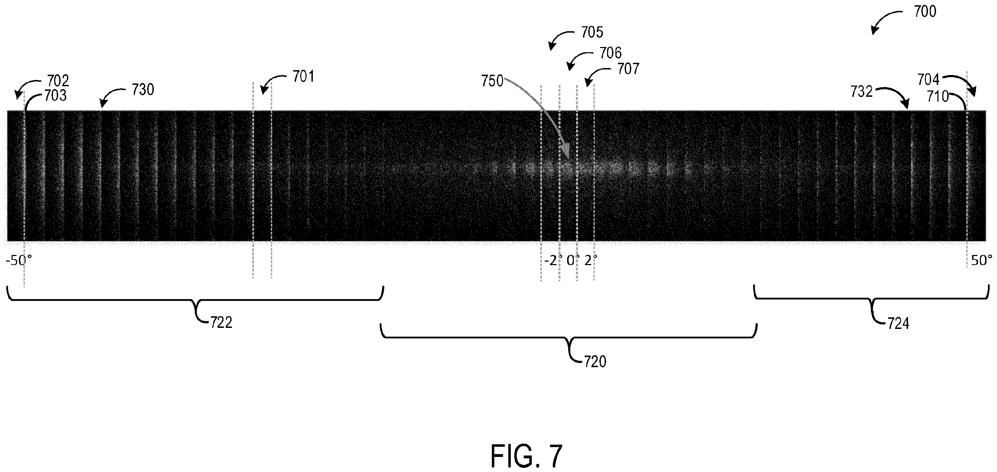

FIG. 7 shows an image 700 of a plurality of projection views 701 of a phantom (e.g., phantoms 302/402) acquired from a detector head (e.g., detector head 408) over a range of sweep angles from -50° to 50°. The projection views 701 are concatenated to facilitate comparison between projection views. In other words, a first projection view 702 of the plurality of projection views at a far left side of image 700 corresponds to a sweep angle of - 50°, and a last projection view 704 of the plurality of projection views at a far right side of image 700 corresponds to a sweep angle of 50°, with projection views acquired at increments of 2° being displayed in between. A projection view 706 corresponds to a sweep angle of 0°, where a field of view of the detector head is aligned with an area of high contrast 750 (e.g., the high contrast region 404) of the phantom. As such, the area of high contrast 750 in a scanned object is most clearly visible in the projection view 706. The area of high contrast 750 is less clearly visible in an adjacent projection view 705, where the detector head is at a sweep angle of -2°, and in an adjacent projection view 707, where the detector head is at a sweep angle of 2°. As the sweep angle decreases towards -50° and increases towards 50°, the visibility of the area of high contrast 750 decreases. At first projection view 702 and second projection view 704, the area of high contrast 750 is not visible, because at the sweep angles of -50° and 50°, the area of high contrast 750 of the phantom is outside the field of view of the detector head.

However, an artifact 730 comprising a vertical penetration of photon counts is visible in a first portion 722 of image 700, which increases as the sweep angle decreases to - 50°. A similar artifact 732 is also visible in a second portion 724 of image 700, which increases as the sweep angle increases to 50°. The artifacts 730 and 732 are not visible in a third, central portion 720 of image 700, where the sweep angle is the smallest and the area of high contrast is in the field of view of the detector head. The artifacts 730 and 732 may be generated by photons that are detected after penetrating shielding of the detector head, as described above in reference to FIGS. 3-5. The increasing intensity of the artifacts 730 and 732 as the sweep angle increases in either the positive or negative direction can be explained by the sweep angle graph 600 of FIG. 6.

Further, the artifacts 730 and 732 are positioned at one side of a respective projection view. That is, the artifact 730 appears at a right side 703 of the projection view 702, and the artifact 732 appears at a left side 710 of the projection view 704. More generally, the artifact 730 may appear at the right side of projection views acquired when the sweep angle is negative, and at the left side of projection views acquired when the sweep angle is positive.

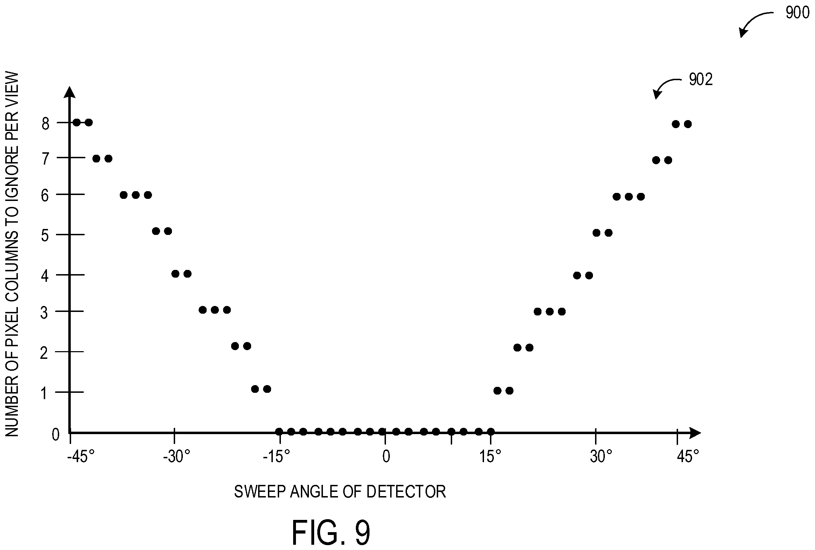

The artifacts 730 and 732 in the wider sweep angle projection views 701 of image 700 may cause corresponding artifacts in an image reconstructed from the projection views. For example, the corresponding artifacts may be visible at a contour of the phantom in the reconstructed image, as shown in FIG. 9. However, because the artifacts 730 and 732 appear at sides of respective projection views (e.g., within columns of pixels positioned at the sides), artifacts 730 and 732 may be advantageously reduced or removed by selectively ignoring one or more side pixel columns of the plurality of projection views 701 when reconstructing an image from the plurality of projection views 701. The selective ignoring may be accomplished by pixel zeroing, described in greater detail below.

In some embodiments, a number of side pixel columns to be selectively ignored may be predefined. In other embodiments, the number of side pixel columns to be selectively ignored may vary across projection views. In at least one embodiment, the number of side pixel columns to be selectively ignored may be selected as a function of a sweep angle of a corresponding detector head, where the number of side pixel columns to be selectively ignored increases as the sweep angle increases. The reduction or removal of the artifacts 730 and 732 using pixel zeroing may be performed in accordance with a method such as method 800 of FIG. 8.

It should be appreciated that while the examples and embodiments described herein refer to selectively ignoring pixel columns, in other embodiments, other groups of adjacent pixels may be selectively ignored without departing from the scope of this disclosure. In other words, in some embodiments, the selectively ignored pixels may not be in a linear profile such as a column. For example, a round profile may be used, or a different shape, where pixels outside or inside the round profile or different shape may be selectively ignored.

Referring now to FIG. 8, a method 800 is shown for increasing a quality of a CT image reconstructed using a SPECT system, such as NM imaging system 100 of FIG. 1A or other embodiments of a SPECT system described herein, by reducing artifacts generated in projection views acquired by the SPECT system as a result of photons that are not collimated at detector heads of the SPECT system, but detected after penetrating shielding positioned around side portions of the detector heads. Method 800 may be performed by a control unit of the SPECT system, such as control unit 130 of FIG. 1A. Method 800 is described with respect to a multi-head SPECT embodiment (e.g., FIG. 2A), where a plurality of detector heads are arranged in a gantry (e.g., gantry 104) that may rotate around a bed or table on which a subject of a scan is placed. However, it should be appreciated that method 800 may be applied to a dual-head SPECT embodiment, where the detector heads are arranged linearly as described in reference to FIGS. 1B and 2B.

Method 800 begins at 802, where method 800 includes performing a scan of an object using the SPECT system. The scanned object may be a patient of the SPECT system, or a phantom, or a different scanned object. The scanned object may include a radioactive tracer, which may emit photons detectable by detectors of the SPECT system. When the scan is performed, a plurality of projection views (e.g., the projection views 701 of FIG. 7) are generated as detector heads of the SPECT system rotate around and sweep across the scanned object. Thus, each projection view may be acquired by a detector head at a corresponding sweep angle of a range of sweep angles of the detector head. In one example, the range of sweep angles may include angles of 0° to 50° in both of a positive and negative direction, as shown in FIG. 7.

At 804, method 800 includes determining, for each projection view, a first portion of pixels that may be used by a reconstruction algorithm of the SPECT system to reconstruct an image volume, and a second portion of pixels including projection data that may not be used by the reconstruction algorithm to reconstruct the image volume. In other words, the reconstruction algorithm may be configured to reconstruct an image based on the projection data of the first portion, and ignore the projection data of the second portion, where the second portion may include an artifact as described above in reference to FIG. 7. For the purposes of this disclosure, ignoring the second portion of pixels or pixel columns refers to ignoring (e.g., not considering) projection data of second portion of pixels or the pixel columns. Ignoring pixel data or pixel columns may be accomplished using a pixel zeroing process, as described in greater detail below. The second portion of pixels to be ignored by the reconstruction algorithm may include pixels located in one or more pixel columns of the projection view. The pixel columns may be positioned at a side of the projection view.

In some embodiments, not all pixels in a pixel column may be ignored. For example, a first portion of pixels of a pixel column may be selectively ignored by the reconstruction algorithm, and a second portion of pixels of the pixel column may not be ignored by the reconstruction algorithm. For example, every other pixel of the pixel column may be selectively ignored, or every third pixel of the pixel column may be selectively ignored, or the pixels of the pixel column to be ignored may be selected in a different manner. As another example, one or more pixel sections within a pixel column may be selectively ignored by the reconstruction algorithm. As yet another example, pixel sections within or outside a defined shape may be selectively ignored.

In some embodiments, the second portion of pixels to be selectively ignored by the reconstruction algorithm may be fixed (e.g., the same) for all of the projection views. For example, the reconstruction algorithm may not be applied to one pixel column of each projection view of the plurality of projection views, or two pixel columns of each projection view of the plurality of projection views, or six columns of each projection view of the plurality of projection views, or a different number of pixel columns. A number of pixel columns (and/or pixels) to be ignored by the reconstruction algorithm may be predefined and stored in a memory of the SPECT system (e.g., the memory 169 of FIG. 1A).

In other embodiments, the second portion of pixels to be selectively ignored by the reconstruction algorithm may not be the same for all of the projection views. For example, a first number of pixel columns of a first set of projection views may be selectively ignored; a second number of pixel columns of a second set of projection views may be selectively ignored; a third number of pixel columns of a third set of projection views may be selectively ignored; and so on. Additionally, a number of pixel columns to be selectively ignored may be determined based on a sweep angle of a detector of a projection view. For example, a first number of pixel columns of a first projection view acquired by a detector at a first sweep angle may be ignored; a second number of pixel columns of a second projection view acquired by the detector at a second sweep angle may be ignored; a third number of pixel columns of a third projection view acquired by the detector at a third sweep angle may be ignored; and so on. The first number, the second number, the third number, etc. may be stored in a lookup table in the memory of the SPECT system.

Further, in some embodiments, the number of pixels and/or pixel columns of the second portion of pixels to be ignored may be determined dynamically and/or adaptively as a function of the sweep angle of a relevant detector, using adaptive pixel zeroing. The function may be represented using a lookup table stored in the memory of the SPECT system, or the function may be stored in the memory of the SPECT system. For example, at an optional step 806, method 800 may include determining a sweep angle of each projection view, and then determining the number of pixels and/or pixel columns of the second portion of pixels to be selectively ignored as a function of the sweep angle. For example, the sweep angle may be an input into the function, and an output of the function may be the number of pixels and/or pixel columns of the second portion of pixels to be selectively ignored.

Referring briefly to FIG. 9, a graph 900 is shown including a plot 902 of an exemplary step function for calculating a number of pixel columns to be ignored by the reconstruction algorithm when reconstructing an image from projection views acquired by detectors at different sweep angles. A number of pixel columns to be ignored per projection view is shown on a vertical axis of graph 900, and a sweep angle of the detector acquiring the projection view is shown on a horizontal axis of graph 900. In accordance with plot 902, between a sweep angle of -15° and a sweep angle of 15°, the first portion of pixels of the projection view used by the reconstruction algorithm to reconstruct an image may include all the pixels of the projection view, and no pixel columns may be ignored by the reconstruction algorithm. At sweep angles of -15° to 15°, photons emitted from a scanned object may be within a field of view of the detector (e.g., corresponding to portion 720 of image 700 of FIG. 7), and no artifacts may be generated in the projection view.

Between a sweep angle of -15° and a sweep angle of -45°, the number of pixel columns to be ignored by the reconstruction algorithm increases linearly from one to eight, as photons emitted from the scanned object are increasingly outside a field of view of the detector, where some photons may penetrate shielding of the detector and cause artifacts in the projection view (e.g., corresponding to portion 722 of image 700). For example, at a sweep angle of -20°, three pixel columns may be ignored by the reconstruction algorithm; at a sweep angle of -30°, four pixel columns may be ignored by the reconstruction algorithm; at a sweep angle of -40°, seven pixel columns may be ignored by the reconstruction algorithm; and so on.

Between a sweep angle of 15° and a sweep angle of 45°, the number of pixel columns to be ignored by the reconstruction algorithm also increases linearly from one to eight, as photons emitted from the scanned object are increasingly outside a field of view of the detector, where some photons may penetrate shielding of the detector and cause artifacts in the projection view (e.g., corresponding to portion 724 of image 700). For example, at a sweep angle of 20°, three pixel columns may be ignored by the reconstruction algorithm; at a sweep angle of 30°, four pixel columns may be ignored by the reconstruction algorithm; at a sweep angle of 40°, seven pixel columns may be ignored by the reconstruction algorithm; and so on.

In this way, the step function illustrated by plot 902 indicates a number of pixel columns to be ignored by the reconstruction algorithm during image reconstruction, to eliminate artifacts generated by the penetrating photons. Additionally, the pixel columns to be ignored may be selected from one side of the projection view, depending on the sweep angle. In particular, the number of pixel columns to be ignored may be adjacent pixel columns at a side of the projection view closest to 0°. For example, in reference to image 700 of FIG. 7, the number of pixel columns to be ignored in projection views 701 for negative sweep angles in portion 722 may be positioned on a right side of a corresponding projection view. The number of pixel columns to be ignored in projection views 701 for positive sweep angles in portion 724 may be positioned on a left side of a corresponding projection view.

Returning to FIG. 8, at 808, method 800 includes generating a mask to apply to each projection view during image reconstruction, based on the pixels or pixel columns to be selectively ignored. The mask may be used to perform pixel zeroing, where pixel data of one or more projection views is converted to values of 0, or values very close to 0 such that the pixel data is ignored by the reconstruction algorithm or has a very small (e.g., negligible) contribution in the calculations of the reconstruction algorithm. In various embodiments, the mask may be a matrix of weight values to be multiplied by a corresponding number of pixels of the projection view, where the weight values are either 1.0 (if the corresponding pixel is to be used by the reconstruction algorithm) or 0.0 (if the corresponding pixel is ignored and not used by the reconstruction algorithm). The matrix may be a two-dimensional (2D) matrix with a length equal to a pixel length of the projection view, and a width equal to a pixel width of the projection view. In some environments, a value such as 0.000001 may be used instead of 0.0.

For example, in one embodiment, the projection view has a length of 279 pixels and a width of 39 pixels. Thus, the projection view includes 39 pixel columns, with each pixel column including 279 pixels. The mask may comprise a 2D matrix of weight values with a length of 279 and a width of 39, such that each weight value of the mask may correspond to (e.g., be multiplied by) a corresponding pixel of the projection view.

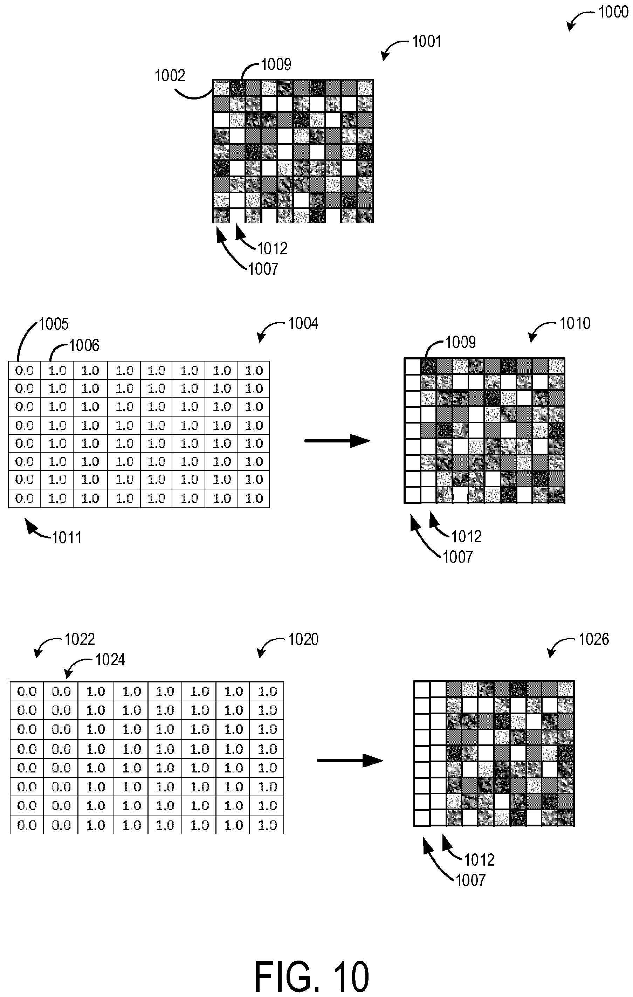

FIG. 10 shows a mask diagram 1000, including depictions of an exemplary projection view 1001 and masks that may be applied to the projection view 1001. For simplicity, in the depicted embodiment, projection view 1001 comprises 10 pixel columns, where each pixel column includes 10 pixels. In other embodiments, projection view 1001 may include a different number of pixel columns (e.g., 39) and/or a different number of pixels per column (e.g., 279). Each pixel may be defined by a pixel intensity value, for example, from 0.0 to 1.0, where 0.0 is black and 1.0 is white.

Mask diagram 1000 includes a first mask 1004 that may be applied to the projection view 1001. First mask 1004 is a 10x10 matrix of weight values, where each weight value corresponds to a pixel of projection view 1001. When the first mask 1004 is applied to the projection view 1001, each weight value of first mask 1004 may be multiplied by the corresponding pixel intensity value of projection view 1001. Thus, a first weight value 1005 of first mask 1004 may be multiplied by a pixel intensity value of first pixel 1002 of projection view 1001. Because the first weight value 1005 is 0.0, the result of multiplying the first weight value 1005 by the pixel intensity value of first pixel 1002 is that the pixel intensity value of first pixel 1002 becomes 0.0. As a result of the pixel intensity value being 0.0, pixel 1002 may be ignored by the reconstruction algorithm when reconstructing an image volume based on the projection view 1001. That is, calculations performed in accordance with the reconstruction algorithm will not include the original pixel intensity value of pixel 1002.

In contrast, a second weight value 1006 of first mask 1004 may be multiplied by a pixel intensity value of second pixel 1009 of projection view 1001. Because the second weight value 1006 is 1.0, the result of multiplying the second weight value 1006 by the pixel intensity value of second pixel 1009 is that the pixel intensity value of second pixel 1009 is unchanged. As a result of the pixel intensity value being unchanged, second pixel 1009 may not be ignored by the reconstruction algorithm when reconstructing an image volume based on the projection view 1001. That is, calculations performed in accordance with the reconstruction algorithm will include the pixel intensity value of second pixel 1009.

First mask 1004 includes a first column 1011 having weight values of 0.0, with other columns of first mask 1004 having weight values of 1.0. As a result, applying first mask 1004 to projection view 1001 results in an adjusted projection view 1010, where pixel intensity values of a first pixel column 1007 are zeroed by first mask 1004. As a result of zeroing the pixel intensity values of first pixel column 1007, pixels of first pixel column 1007 may be ignored by the reconstruction algorithm during image reconstruction, while pixel intensity values of pixels of the other pixel columns of the projection view 1001 may be used by the reconstruction algorithm to create the image volume.

Mask diagram 1000 includes a second mask 1020 that may be applied to the projection view 1001, which may be used to zero pixel intensity values of both the first pixel column 1007 and a second column 1012 of the projection view 1001. The second mask 1020 includes a first column 1022 of weight values of 0.0 and a second column 1024 of weight values of 0.0, with remaining columns having a weight value of 1.0. Applying the second mask 1020 to the projection view 1001 results in an adjusted projection view 1026, where pixels of both the first pixel column 1007 and the second column 1012 of the adjusted projection view 1026 may be ignored by the reconstruction algorithm during image reconstruction, and pixel intensity values of pixels of the other pixel columns of the adjusted projection view 1026 may be used by the reconstruction algorithm to create the image volume. In this way, different masks may be configured that when applied to a projection view, result in various pixel columns of the projection view being ignored by the reconstruction algorithm. In some embodiments, for each projection view, a same mask may be used to ignore a same number of pixel columns, while in other embodiments, a different mask may be generated for different pixel columns based on the sweep angle of the relevant detector (e.g., adaptive pixel zeroing).

Returning to FIG. 8, at 810, method 800 includes reconstructing an image (e.g., image volume) from the projection views acquired during the scan, where corresponding masks may be applied to projection views to reduce artifacts generated by photons penetrating detector shielding. In particular, the masks may be applied based on a sweep angle of a detector associated with a projection view, using a lookup table and/or function such as the function illustrated in FIG. 9. Various methods may be used to ignore pixels or full views during the reconstruction process. An iterative reconstruction algorithm may be used, such as ordered subset expectation maximization (OSEM) or block-sequential regularized expectation maximization (BSREM), or a different reconstruction algorithm may be used. In various embodiments, the reconstruction algorithm may include a pixel weight input, corresponding to a matrix of weights for each pixel. Prior to or during reconstruction, each pixel weight may be multiplied by a corresponding pixel value. Thus, pixels that are assigned pixel weights of zero or very low pixel weights (e.g., close to zero) may be ignored (e.g., may have negligible contributions) by the reconstruction algorithm. The pixel weights may be assigned based on various factors, such as the view acquisition time and/or other correction methods.

At 812, method 800 includes displaying the reconstructed image on a display device of the SPECT system (e.g., the display device 168 of FIG. 1A), and method 800 ends. Additionally or alternatively, the reconstructed image may be stored in a memory of the SPECT system (e.g., the memory 169 and/or data storage device 164). When the reconstructed image is displayed on the display device, artifacts present in the projection views acquired by the SPECT system may not appear in the reconstructed image, and a quality of the reconstructed image may be increased.

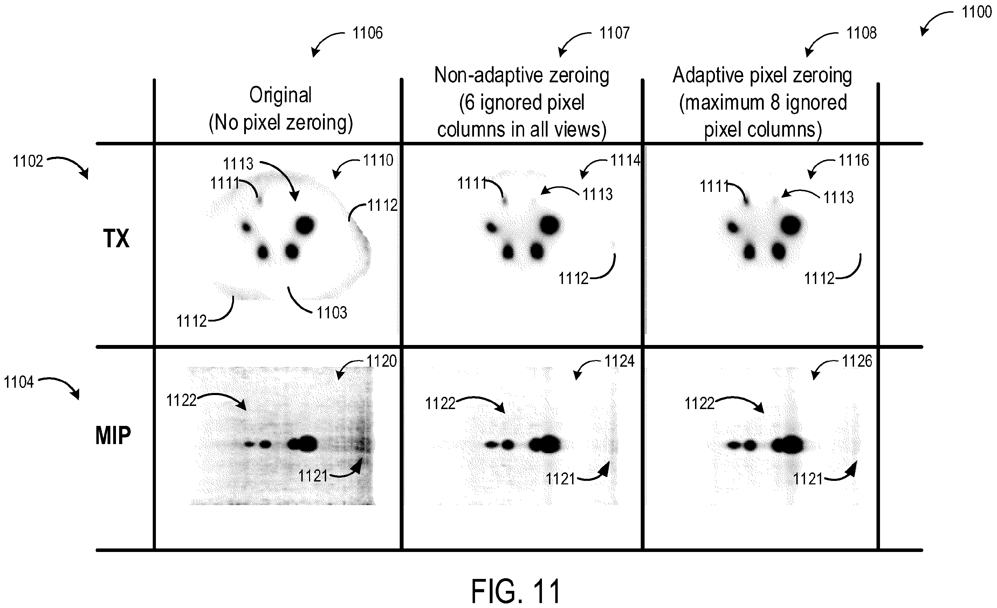

FIG. 11 shows a table 1100 including six examples of reconstructed images generated from a phantom 1103 by the SPECT system where pixel zeroing is advantageously used to configure a reconstruction algorithm of the SPECT system to ignore pixels of projection views including artifacts. The phantom 1103 includes 6 high contrast regions of varying sizes and at different locations within the phantom 1103, shown in black in the images, which include a radioactive tracer that emits photons detectable by the SPECT system.

Table 1100 includes three columns. A first column 1106 shows reconstructed images where no pixel zeroing is used in image reconstruction. A second column 1107 shows reconstructed images where non-adaptive pixel zeroing is used in image reconstruction, where six pixel columns of each projection view including artifacts are ignored by the reconstruction algorithm. A third column 1108 shows reconstructed images where adaptive pixel zeroing is used in image reconstruction, where a varying number of from one to eight pixel columns of each projection view including artifacts are ignored by the reconstruction algorithm, in accordance with a function such as the function depicted in FIG. 9.

The table 1100 includes two rows. A first row 1102 shows transaxial (TX) views of the reconstructed images, and a second row 1104 shows a coronal view of the reconstructed images, where the coronal view is generated using maximum intensity projection (MIP).

In the first column 1106, a first transaxial image 1110 is shown where no pixel zeroing is used. Transaxial image 1110 shows five out of the six high contrast regions, but a smallest high contrast region 1113 is not visible. Additionally, a next-smallest high contrast region 1111 is blurry, and not displayed in high contrast. Artifacts 1112 due to penetrating photons are visible around a contour of phantom 1103. Similarly, a first MIP image 1120 shows artifacts 1121 (at a side of MIP image 1120) and 1122 (streaking).

In the second column 1107, a second transaxial image 1114 is shown, where the non-adaptive pixel zeroing is used, and six pixel columns are ignored by the reconstruction algorithm using masks as described above in reference to FIG. 10. In contrast with first transaxial image 1110, all six high contrast regions are visible in transaxial image 1114, but the smallest high contrast region 1113 is faint. A contrast of the next-smallest high contrast region 1111 is increased, but still blurry. The artifacts 1112 are not as visible around the contour of phantom 1103, although still present. Thus, as a result of the non-adaptive pixel zeroing, a quality of second transaxial image 1114 is greater than a quality of first transaxial image 1110. A second MIP image 1124 similarly shows a decreased visibility of artifacts 1121 and 1122 with respect to the first MIP image 1120.

In the third column 1108, a third transaxial image 1116 is shown, where the adaptive pixel zeroing is used, in accordance with a function such as the function plotted in FIG. 9. For example, a lesser number of pixel columns may be zeroed at a lower detector sweep angles, and a higher number of pixel columns may be zeroed at higher detector sweep angles, where the number of pixel columns that are zeroed range from one to eight. In third transaxial image 1116, all six high contrast regions are visible, and the contrast of the smallest high contrast region 1113 and the next-smallest high contrast region 1111 are increased with respect to first transaxial image 1110 and second transaxial image 1114. The artifacts 1112 around the contour of phantom 1103 are almost completely eliminated. Thus, as a result of the adaptive pixel zeroing, a quality of third transaxial image 1116 is greater than both of second transaxial image 1114 and first transaxial image 1110. A third MIP image 1126 shows a similar reduction in the visibility of artifacts 1121 and 1122 with respect to the first MIP image 1120 and the second MIP image 1124.

Thus, a method for removing artifacts due to photons that penetrate detector shielding from an image reconstructed by a SPECT system is disclosed. Because the artifacts appear at a side of projection views acquired by the SPECT system, the artifacts may be reduced or eliminated in the reconstructed image by configuring a reconstruction algorithm to reconstruct the image based on a first portion of pixels not including the artifacts of each projection view, and to ignore a second portion of pixels including the artifacts of the projection view. The second portion of pixels may include one or more adjacent pixel columns located at a side of a projection view. A size of the second portion of pixels (e.g., a number of pixel columns) to be ignored may depend on a sweep angle of a detector acquiring the projection view. At low sweep angles close to 0°, artifacts due to penetrating photons may not appear in a projection view, and no pixel columns may be ignored. As the sweep angle increases farther from 0° and the artifacts increase in size, the reconstruction algorithm may be configured to ignore an increasing number of pixel columns. The number of pixel columns to be ignored may be determined as a function of detector sweep angle, for example, a step function, which may be represented in a lookup table of the SPECT system. The reconstruction algorithm may be configured to ignore the pixel columns by applying a mask to some or all of the projection views used to reconstruct the image to convert pixel intensity values of pixels of the pixel columns to zero, such that the pixel columns do not contribute towards the generation of the reconstructed image. In this way, the reconstructed image may not include the artifacts, and an overall quality of the reconstructed image may be increased. By increasing the overall quality of the reconstructed image, a radiologist may more quickly and efficiently detect abnormalities in the reconstructed image and a diagnosis of the radiologist may be more accurate, resulting in improved patient treatment. The technical effect of configuring a reconstruction algorithm of a SPECT system to ignore portions of pixels of projection views when reconstructing an image is that artifacts caused by non-collimated photons that penetrate detector shielding may be reduced, generating a higher quality reconstructed image.

The disclosure also provides support for a method for a single photon emission computed tomography (SPECT) imaging system, the method comprising: acquiring a plurality of projection views of a scanned object using the SPECT imaging system, the plurality of projection views including artifacts, configuring a reconstruction algorithm to reconstruct an image based on a first portion of pixels of each projection view of the plurality of projection views, and not based on a second portion of pixels of each projection view of the plurality of projection views, the second portion of pixels including the artifacts, reconstructing an image using the reconstruction algorithm, and displaying the reconstructed image on a display device of the SPECT imaging system and/or storing the reconstructed image in a memory of the SPECT imaging system. In a first example of the method, the SPECT imaging system is a multi-head SPECT imaging system comprising a plurality of detector heads arranged on a gantry around the scanned object, and the plurality of projection views includes projection views acquired at different sweep angles of each detector head. In a second example of the method, optionally including the first example, configuring the reconstruction algorithm to reconstruct the image based on the first portion of pixels and not based on the second portion of pixels further comprises applying a mask to a projection view when applying the reconstruction algorithm to cause the reconstruction algorithm to ignore the second portion of pixels of the projection view, wherein the mask is a matrix of weights to be multiplied by each pixel of the projection view, the matrix of weights including weights of 1.0 for pixels to be used by the reconstruction algorithm and weights of 0.0 for pixels to be ignored by the reconstruction algorithm. In a third example of the method, optionally including one or both of the first and second examples, the second portion of pixels to be ignored by the reconstruction algorithm comprises one or more columns of pixels. In a fourth example of the method, optionally including one or more or each of the first through third examples, the projection data of the second portion of pixels to be ignored by the reconstruction algorithm comprises projection data of pixels outside or inside a defined shape. In a fifth example of the method, optionally including one or more or each of the first through fourth examples, a number of the one or more pixel columns to be ignored by the reconstruction algorithm is fixed for the plurality of projection views. In a sixth example of the method, optionally including one or more or each of the first through fifth examples, a number of the one or more pixel columns to be ignored by the reconstruction algorithm varies across the plurality of projection views. In a seventh example of the method, optionally including one or more or each of the first through sixth examples, the number of the one or more pixel columns of a projection view to be ignored by the reconstruction algorithm varies as a function of a sweep angle of a detector acquiring the projection view. In an eighth example of the method, optionally including one or more or each of the first through seventh examples, a number of the one or more pixel columns to be ignored is retrieved from a lookup table stored in the memory of the SPECT imaging system. In a ninth example of the method, optionally including one or more or each of the first through eighth examples, the artifacts are generated by photons penetrating a shielding of a detector of the SPECT imaging system. In a tenth example of the method, optionally including one or more or each of the first through ninth examples,: in a first condition, wherein the reconstruction algorithm is not configured to reconstruct the image based on the first portion of pixels of each projection view of the plurality of projection views and not based on the second portion of pixels of each projection view of the plurality of projection views, and the artifacts are visible in the reconstructed image, and in a second condition, wherein the reconstruction algorithm is configured to reconstruct the image based on the first portion of pixels of each projection view of the plurality of projection views and not based on the second portion of pixels of each projection view of the plurality of projection views, and the artifacts are not visible in the reconstructed image.

The disclosure also provides support for a single photon emission computed tomography (SPECT) imaging system, comprising: a plurality of detector heads, each detector head including shielding arranged around a detector of the detector head, a controller including instructions stored in a memory of the SPECT imaging system, that when executed, cause the controller to: acquire a plurality of projection views of a scanned object using the SPECT imaging system, partially or completely remove artifacts generated in one or more projection views of the plurality of projection views, the artifacts caused by photons penetrating the shielding, apply a reconstruction algorithm to the plurality of projection views to reconstruct an image, and display the reconstructed image on a display device of the SPECT imaging system and/or storing the reconstructed image in the memory. In a first example of the system, further instructions are stored in the memory that when executed, cause the controller to apply a mask to the one or more projection views during image reconstruction to cause the reconstruction algorithm to ignore one or more pixel columns of the one or more projection views including the artifacts, wherein the mask is a matrix of weights to be multiplied by each pixel of a projection view, the matrix of weights including weights of 1.0 for pixels to be considered by the reconstruction algorithm and weights of 0.0 or close to 0.0 for pixels to be ignored by the reconstruction algorithm. In a second example of the system, optionally including the first example, the one or more ignored pixel columns are adjacent pixel columns located at one side of a projection view. In a third example of the system, optionally including one or both of the first and second examples, a number of the one or more ignored pixel columns is fixed for the one or more projection views. In a fourth example of the system, optionally including one or more or each of the first through third examples, a number of the one or more ignored pixel columns of a projection view varies as a function of a sweep angle of a detector that acquired the projection view. In a fifth example of the system, optionally including one or more or each of the first through fourth examples, the number of the one or more ignored pixel columns is between one and eight. In a sixth example of the system, optionally including one or more or each of the first through fifth examples, as a result of partially or completely removing the artifacts generated in the one or more projection views, an image quality of the reconstructed image is increased.

The disclosure also provides support for a method for increasing a quality of an image generated by a single photon emission computed tomography (SPECT) imaging system, the method comprising: acquiring a plurality of projection views of a scanned object using the SPECT imaging system, determining a number of pixel columns of one or more projection views of the plurality of projection views in which an artifact is visible, the artifact a result of photons penetrating shielding of a detector of the SPECT imaging system, applying a mask to the one or more projection views to convert pixel intensity values of the pixel columns to zero, applying a reconstruction algorithm to the plurality of projection views to reconstruct an image not including the artifact, and displaying the reconstructed image on a display device of the SPECT imaging system and/or storing the reconstructed image in a memory of the SPECT imaging system. In a first example of the method, the number of pixel columns in a projection view of the plurality of projection views is based on a detector sweep angle of the projection view.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A method (800) for a single photon emission computed tomography (SPECT) imaging system, the method (800) comprising:
acquiring a plurality of projection views of a scanned object using the SPECT imaging system (802), the plurality of projection views including artifacts;
configuring a reconstruction algorithm to reconstruct an image based on projection data of a first portion of pixels of each projection view of the plurality of projection views, and ignore projection data of a second portion of pixels of each projection view of the plurality of projection views, the second portion of pixels including the artifacts;
reconstructing an image using the reconstruction algorithm (810); and
displaying the reconstructed image on a display device of the SPECT imaging system and/or storing the reconstructed image in a memory of the SPECT imaging system (812).

2. The method (800) of claim 1, wherein the SPECT imaging system is a multi-head SPECT imaging system comprising a plurality of detector heads arranged on a gantry around the scanned object, and the plurality of projection views includes projection views acquired at different sweep angles of each detector head.

3. The method (800) of claim 1, wherein configuring the reconstruction algorithm to reconstruct the image based on the projection data of the first portion of pixels and ignore the projection data of the second portion of pixels further comprises applying a mask to a projection view when applying the reconstruction algorithm (808), wherein the mask is a matrix of weights to be multiplied by each pixel of the projection view, the matrix of weights including weights of 1.0 for pixels to be used by the reconstruction algorithm and weights of 0.0 or close to 0.0 for pixels to be ignored by the reconstruction algorithm.

4. The method (800) of claim 1, wherein the projection data of the second portion of pixels to be ignored by the reconstruction algorithm comprises projection data included in one or more columns of pixels located at a side of a projection view.

5. The method (800) of claim 1, wherein the projection data of the second portion of pixels to be ignored by the reconstruction algorithm comprises projection data of pixels outside or inside a defined shape.

6. The method (800) of claim 4, wherein a number of the one or more pixel columns to be ignored by the reconstruction algorithm is fixed for the plurality of projection views.

7. The method (800) of claim 4, wherein a number of the one or more pixel columns to be ignored by the reconstruction algorithm varies across the plurality of projection views.

8. The method (800) of claim 7, wherein the number of the one or more pixel columns of a projection view including projection data to be ignored by the reconstruction algorithm varies as a function of a sweep angle of a detector acquiring the projection view.

9. The method (800) of claim 4, wherein a number of the one or more pixel columns including projection data to be ignored is retrieved from a lookup table stored in the memory of the SPECT imaging system.

10. The method (800) of claim 1, wherein the artifacts are generated by photons penetrating a shielding of a detector of the SPECT imaging system.

11. The method (800) of claim 1, wherein the artifacts are not visible in the reconstructed image.

12. A single photon emission computed tomography (SPECT) imaging system (200, 100), comprising:
a plurality of detector heads (308, 408, 210), each detector head (308, 408, 210) including shielding (580) arranged around a detector (174, 474) of the detector head (308, 408, 210);
a controller including instructions stored in a memory (169) of the SPECT imaging system (200, 100), that when executed, cause the controller to:
acquire a plurality of projection views (701) of a scanned object using the SPECT imaging system (200, 100);
partially or completely remove artifacts (1121, 1112, 730) generated in one or more projection views (701) of the plurality of projection views (701), the artifacts (1121, 1112, 730) caused by photons penetrating the shielding (580);
apply a reconstruction algorithm to the plurality of projection views (701) to reconstruct an image (700); and
display the reconstructed image (1114, 1116) on a display device (168) of the SPECT imaging system (200, 100) and/or storing the reconstructed image (1114, 1116) in the memory (169).

13. The SPECT imaging system (200, 100) of claim 12, wherein further instructions are stored in the memory (169) that when executed, cause the controller to:
apply a mask (1004, 1020) to the one or more projection views (701) during image reconstruction to cause the reconstruction algorithm to ignore one or more pixel columns (1007, 1012) of the one or more projection views (701) including the artifacts (1121, 1112, 730), wherein the mask is a matrix of weights to be multiplied by each pixel (1002) of a projection view (701), the matrix of weights including weights of 1.0 for pixels to be considered by the reconstruction algorithm and weights of 0.0 for pixels to be ignored by the reconstruction algorithm.

14. The SPECT imaging system (200, 100) of claim 13, wherein the one or more ignored pixel columns (1007, 1012) are adjacent pixel columns located at one side of a projection view (701).

15. The SPECT imaging system (200, 100) of claim 13, wherein a number of the one or more ignored pixel columns (1007, 1012) is fixed for the one or more projection views (701).
